# EUROPEAN PATENT APPLICATION

(11) **EP 3 034 060 A1**
(43) Date of publication of application: **22.06.2016**
(21) Application number: 14425156.8
(22) Date of filing: 19.12.2014
(51) Int. Cl.: A61H 1/00, A61H 23/04, A61N 5/06

(54) **PNEUMATIC MASSAGE BED**

(30) Priority: 17.12.2014 IT RM20140732
(71) Applicant: ASIAGEM di Nicoletti Tommaso, 73048 Nardo (LE) (IT)
(72) Inventor: Tommaso, Nicoletti, 73052 Parabita (LE) (IT)
(74) Representative: De Tullio, Michele Elio

(57) **Abstract**

The pneumatic massage bed is characterized by pneumatic actuators operable instantaneously and individually, or grouped in clusters, or in a mixed manner, individually and in clusters.

The operation mode of said pneumatic actuators can be activated and controlled via a dedicated touch interface, installable on wireless, or alternatively wired, devices, in an automatic or customized manner, effecting linear and / or circular waves, triggered respectively on the screen of a mobile device that represents the massage bed by typing a vector or touch with a finger in a predetermined point.

## Description

The present invention relates to a bed that permits benefic effects and rest, which implements ergonomic functions and massaging effects.

More particularly it relates to a horizontal base, commonly said bed base, comprising a pneumatic system, consisting of cylinders and pistons, and a suitable mattress, for ergonomic support and massage of the user who uses the bed; the pneumatic cylinders/pistons system can be activated by a touch interface installable on wireless, or alternatively wired, devices.

Massage beds and mattresses are currently used as benefic devices or medical facilities for treating specific diseases related to postural problems, mostly of the spine. In particular, among the commonly known applications there are the following:
- for the rest of the user,
- to perform benefic effects or therapeutic massage,
- to boost bloodstream by warming effect.

To perform these treatments the massage beds and mattresses are mostly provided with fluid systems (pneumatic of hydraulic) for ergonomic support and/or active or passive massage of the user.

In particular, to perform the massaging effect, said massage beds and mattresses are provided with mechanical systems comprising a series of vertical cylinders and pistons supporting, in the upper part of the stem, suitable heads, or alternatively, the slats of the bed base, that contact the user.

For their operation, these cylinder/piston mechanisms, are supplied by a hydraulic or compressed air source, and are provided at the bottom with interconnected rooms adapted also to balance the weight of the user resting or lying on the bed.

There are also mattresses or beds which are provided, as well as with means for effecting massage, even with devices which implement a warming effect on the user, for example by means of infrared sources, and a therapeutic effect, for example by means of stones, such as jade or tourmaline.

In the state of the art, are known several massage beds and mattresses provided with fluid systems (pneumatic or hydraulic) for ergonomic support and/or active or passive massage of the user; some of them are illustrated in some patent documents, among which are cited the following:
- KR20090076148 entitled "Bedsore prevention mattress" filed on 18.08.2009,
- DE3728408 entitled "B" filed on 08.26.1997,
- DE3615421 entitled "Massaging bed" filed on 07.05.1986,
- CN2376903 entitled "Pneumatic massage bed" filed on 21.05.1999,
- CN202288754 entitled "Pneumatic massage bed" filed on 02.10.2011,
- CN201551530 entitled "Inflation massage pad" filed on 21.12.2009,
- CN2086549 entitled "Health-care massage mattress" filed on 18.04.1991,
- CN200960275 entitled "Multifunctional thermal effect health massage pad" filed on 30.10.2006.

From a detailed analysis of the above identified patent documents, and in particular of the documents KR20090076148, DE3728408, and CN200960275 it is clear that most of the beds and mattresses provided with massaging pneumatic fluid systems are constituted by:
- a plurality of cylinders each one having a vertical body, a piston engaged in each body of the cylinder provided with a member engaged at an upper end which contact the back of the user,
- a device for supplying high pressurized air to the pneumatic cylinders,
- air supply tubes that connect the pneumatic cylinders to the air supply unit,
- a control unit with manages the operations of the apparatus of air supply apparatus and the pneumatic cylinders.

In additions, the basic idea of the pneumatic or hydraulic operated massage beds according of the aforementioned state of the art is based on the fulfillment of the following features and applications:
- for the rest of the user, passive use of the pistons,
- to perform massage, active use of the pistons,
- warming and therapeutic treatment by means of infrared sources and similar, stones having relaxing effect.

Instead, the massage bed according to the present invention combines, beyond the above mentioned known main features and applications, a further feature and application not emerged in the aforementioned documents of the state of the art.

In particular, the possibility for the user to operate one or more pistons (single or by groups) of the pneumatic system implemented in the horizontal base of the bed by means of a touch interface installable on a mobile device (smartphone, tablet, etc.), according to the direction of linear and circular waves that, propagating through the mattress, have an impact on the user.

The use of said touch interface, to operate the massaging effect, gives a new effectiveness and ease of the use of known beds and mattresses for massage.

The main advantages are due to the fact that the operation of the pneumatic massaging system would become instant, easy and customizable according to the needs of the user.

In the pneumatic drive system according to the invention, further devices are provided, described in detail below, which allow an improvement of the massaging effect on the user or alternatively for his rest.

In particular, by the operation of single pistons or groups of pistons, it is possible to perform a massaging effect differentiable according to requirements.

The improvement of the user's rest is obtained instead by means that allow an ergonomic adjustment of the mattress to his anatomical structure, based on the operation of pistons by communicating vessels; the effect achieved is a homogeneous distribution of the user's weight on the bed giving the feeling to be suspended.

Further advantages and features of the invention will become apparent from the following description of an embodiment, being purely illustrative and therefore not limiting, of a pneumatic massage and relevant operation mode described with reference to the accompanying drawings that illustrate, respectively:
figure 1 - an axonometric view of the massage bed without mattress;
figure 2 - an axonometric view from below of the pneumatic system of the massage bed consisting of clusters, four pistons each, arranged at the vertices of a square;
figure 3 - an axonometric view of one of the square clusters of figure 2;
figure 4 - the movements of each piston during his operation;
figure 5 - the movements of the mobile head of each piston;
figure 6 - a front view and a cross section A-A of one piston;
figure 7 - an exploded axonometric view of the piston of the preceding figures;
figure 8 - the components of each piston;
figure 9 - a schematic view of the input on a tablet to operate the pistons effecting linear massaging waves;
figure 10 - a schematic view of the propagation of a linear massaging wave;
figure 11 - a schematic view of the input on a tablet to operate the pistons effecting circular massaging waves;
figure 12 - a schematic view of the propagation of a circular massaging wave;
figure 13 - schematic views of the movements of some square clusters, illustrated in figures 2 and 3, operated respectively by horizontal (a), diagonal (b), vertical (c) massaging waves;
figure 14 - schematic views of the movement of some clusters, each of three pistons, arranged at the vertices of a triangle, operated respectively by horizontal (a), diagonal (b), horizontal broken (c) massaging waves;
figure 15 - some schematic views of the movement of single pistons, or grouped by clusters in horizontal segments, operated respectively by horizontal (a) and diagonal (b) massaging waves;

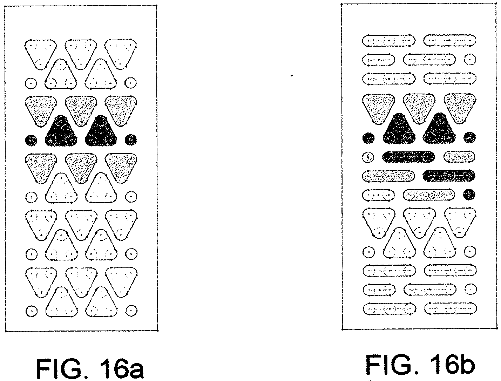

figure 16 - a schematic view of the hybrid movement respectively of single pistons and grouped in a triangle (a), and of single pistons and grouped by clusters in horizontal segments and grouped in a triangle (b).

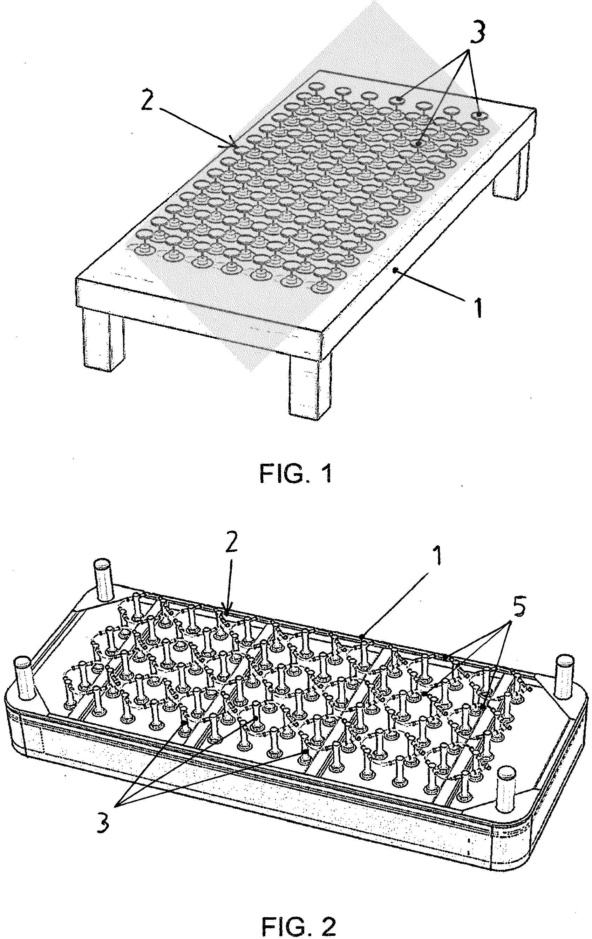

The massage bed, according to the present invention, includes a horizontal base 1 shown in figure 1, commonly said bed base provided with a pneumatic system 2 and with a suitable mattress for the ergonomic support, rest and massage of the user who uses the bed.

The pneumatic system 2 implemented in the horizontal base 1, shown in an axonometric view from below in figure 2, is activated through the use of compressed air and it is constituted by a series of pneumatic actuators, or single-acting pistons 3, arranged in such a way to cover uniformly the central area 1 of the bed base on which one is resting on the mattress.

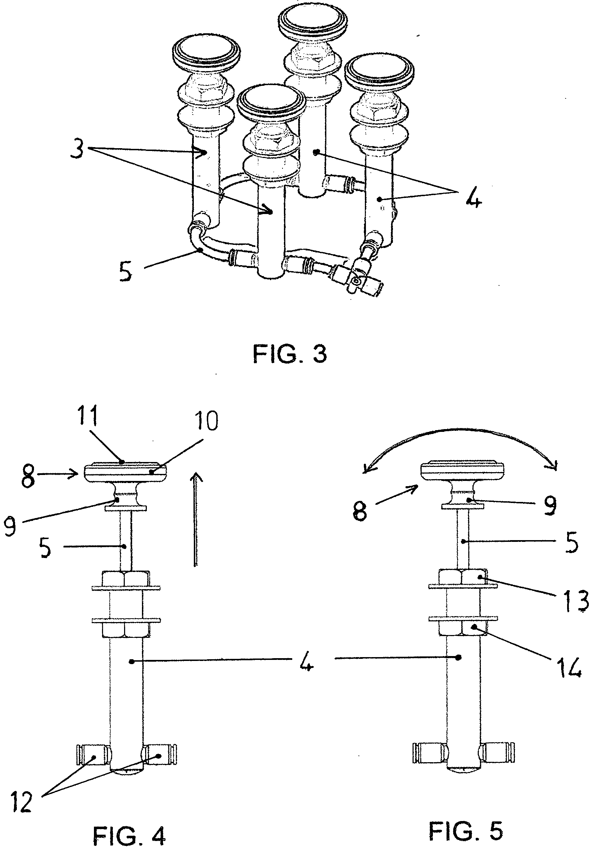

Said pistons 3 are actuated in cluster groups, for example, four pistons each, arranged at the vertices of a square (figure 3) or other polygonal shapes, or alternatively sorted in rows or columns or broken lines, or mixed groups of said configurations; each cluster has at the base of the pistons a supply line (pressurized air) in common, which determines their simultaneous actuation.

Each piston 3 has a single chamber, consisting of a hollow cylindrical body 4, inside of which flows a stem5; the strength of each single piston is developed in single direction from the bottom to the top (figure 4).

The final stage of stem extension and return of the same towards its operation direction is cushioned by means of spring device 20, inserted inside the cylinder 4 coaxially with said stem 5 and in abutment with a movable head plunger 6 provided with a soft seal 7 and connected to the lower end of the stem 5; the piston is repositioned by the action of the force exerted by user's weight lying on the massage bed.

The upper end of the stem 5 of the piston is elastically connected to a movable head 8 by means of a rubber ball joint9 that allows the inclination of the head in all directions (figure 5) to fit to the anatomical shapes of the user who uses the bed.

A flat circular surface 10, connected by suitable means, such as screw, to said rubber joint 9 allows the application, for example by gluing, of disks 11 of suitable material.

In other words, said flat surface 10 provides a flat holder for the support of a disk of stone, such as jade, tourmaline, possibly heated or illuminated with LEDs or alternatively with infrared rays, which in contact with the user's body determines further benefic effect allowing muscular loosen and relax, while reactivating the bloodstream with a therapeutic vaso-dilating effect.

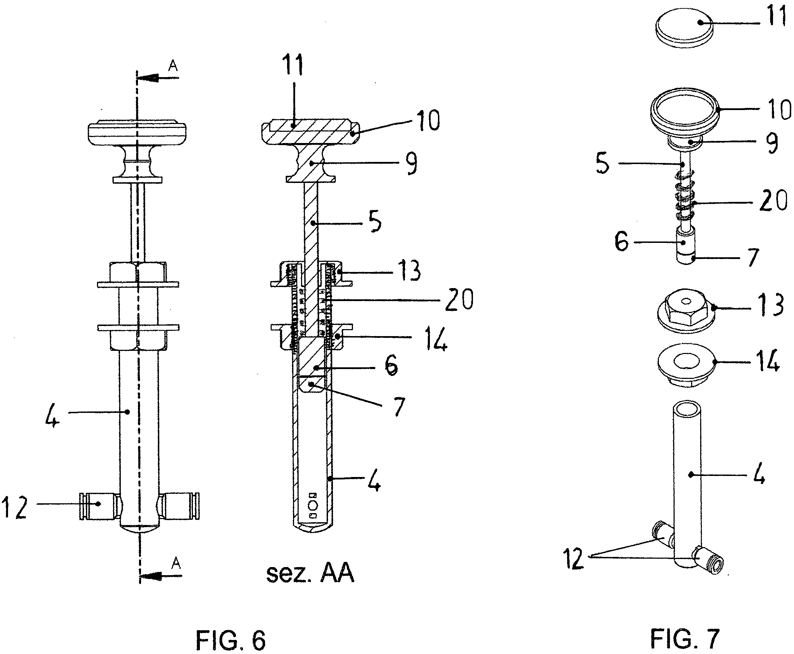

One piston of the pneumatic system of the massaging mattress is shown in detail in a front view and a cross-section A-A in figure 6 and in an exploded axonometric view in figure 7.

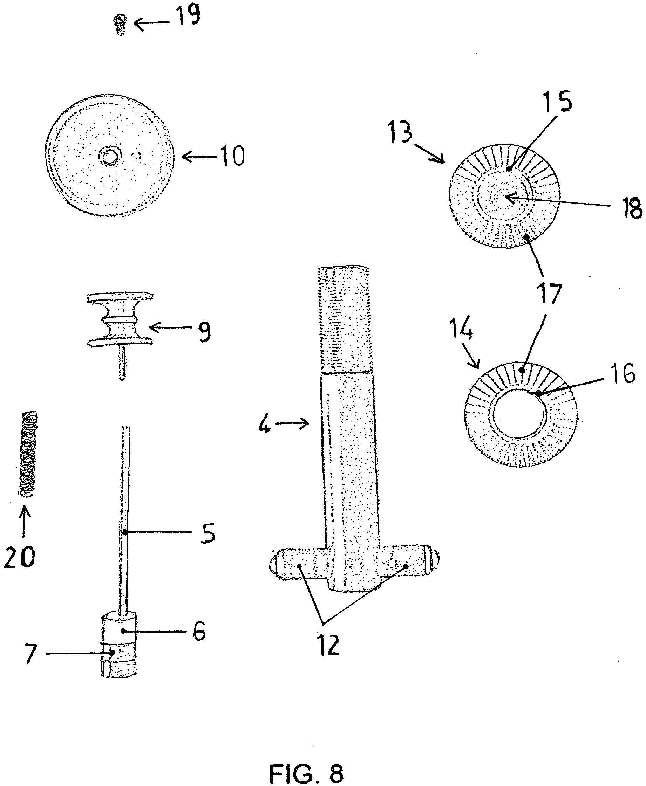

As illustrated in detail in figure 8, the cylindrical body 4 of the piston integrates at the bottom two fast connectors 12, hot plugged, for the connection of suitable compressed air tubes.

Two ferrules, an upper 13 and a lower 14, screwed by means of threads 15, 16 on the hollow cylindrical body 4 of the piston allow the fixing of the piston on panels of several thickness; for this purpose, the support faces of said ferrules are provided with grooves 17 preventing unscrewing.

The upper ferrule 13, acting as a cap for the piston body, is provided with trough hole 18 for the sliding of the piston stem 5.

Above the piston is provided the application of a thin layer (for example foam of bamboo or alternatively latex, natural polymers, viscoelastic materials), which gives a nearly uniform surface, similar to that of a mattress, for the user's support lying.

At the bottom of the pistons are disposed infrared sources equipped with disks in stone (ex. tourmaline) for the heating of the mat which, provided with through holes, allows the exchange of heat between the massage bed and the user.

In order to produce a massaging effect, the user can control the operation of the pistons individually or in groups.

In particular, via a dedicated touch interface, usable on mobile devices such as smartphones, tablets, or similar devices, the massaging effect is activated automatically or customized according to the direction of linear or circular waves that, propagating through the mattress, have an impact on the user.

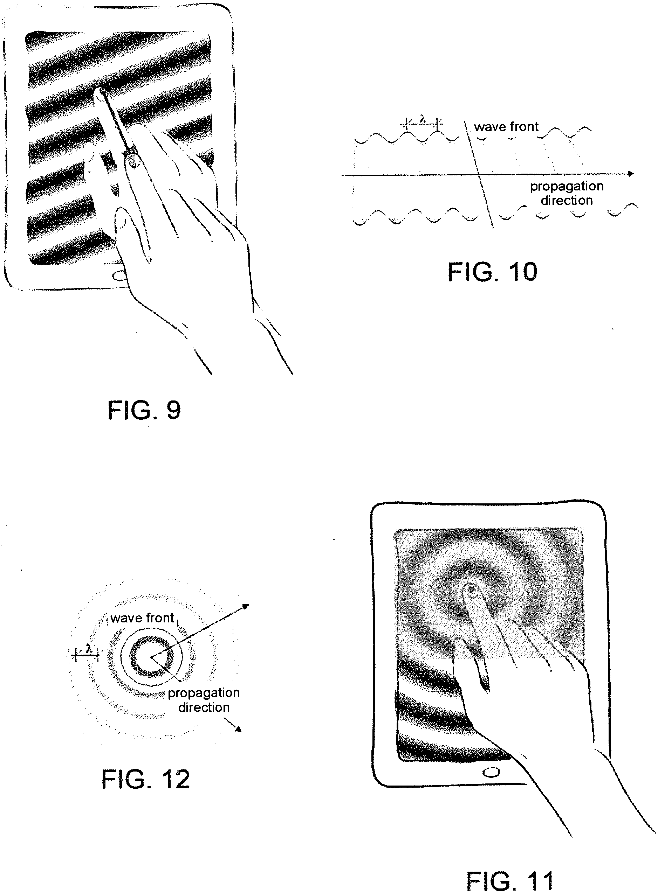

More in detail, as illustrated in the schematic views in figures 9 and 10, to trigger a linear wave massage the user will type a vector on the screen of the mobile device that represents the massage bed; the linear wave massage will be triggered in the direction indicated by the vector and with a speed proportional to the performed typing speed. To trigger instead a circular wave massage, as illustrated in the schematic views in figures 11 and 12, the user will touch on the screen on the interface, the trigger point from which the circular wave will have to generate.

The pneumatic massage bed and relevant operation mode according to the present invention allows several improvement of the currently known applications in the following considerations.

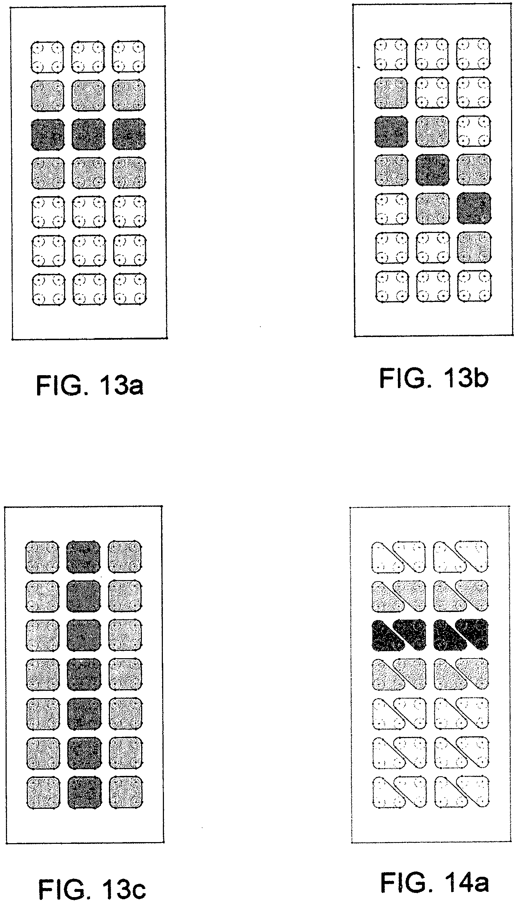

A first improved application is obtained in the use of the massage bed for the rest of the user (passive use of the pistons): moving heads of the pistons below the mattress, fit the anatomical shape structure of the user, facilitating the operation of pistons in their cylinders by communicating vessels, and the effect of homogeneous distribution of pressure of the user's weight on the bed giving the feeling of being suspended. Another improved application is the use of the massage bed to perform massages (active use of pistons): as shown in the figures 13, 14, 15 ad 16 the pistons, activated by the use of compressed air, can be operated individually or in groups, creating a different massage effect.

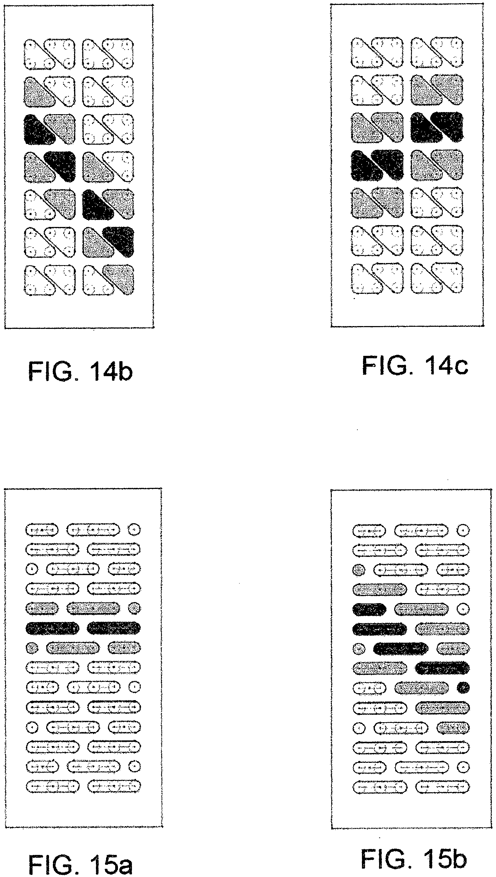

In particular it is possible to implement massages by pressing some square clusters , figure 13, operated respectively by horizontal 13a, diagonal 13b, vertical 13c massaging waves or alternatively cluster groups, each one of three pistons, arranged at the vertices of a triangle, figure 14, operated respectively by horizontal 14a, diagonal 14b, horizontal broken 14c massaging waves or single pistons or grouped by a cluster in horizontal segments, figure 15 operated respectively by horizontal 15a and diagonal 15b massaging waves, or for mixed clusters of single pistons and grouped in a triangle 16a, and single pistons grouped by cluster in horizontal segments and grouped in a triangle 16b.

The massaging mattress, compared to similar devices of the prior art, allows an improvement of the heating and therapeutic effects:
- the infrared sources arranged at the bottom of each piston produce dry heat treatment which, in contact with the user's body, gives a general benefic effect;
- the through holes of the mattress facilitate the heat exchange between the user and the massage bed;
- the stone disks, such as in jade or tourmaline, have therapeutic properties, solving problems related to the bone, immune, circulatory system and rebalancing for the whole organism.

The present invention has been described for illustrative but not limitative purpose, but it has to be understood that variations or modifications may be made by men skilled in the art without departing from the relative scope of protection, as defined by the attached claims.

## Claims

1. "Pneumatic massage bed and relevant operation mode" comprising a horizontal base (1) provided with a pneumatic system (2) and a suitable mattress, said pneumatic system comprising:
• a series of pneumatic actuators (3), arranged in order to cover uniformly the central area of the horizontal base (1) on which said mattress is supported;
• a device for supplying pressurized air to said pneumatic actuators (3);
• air supply tubes that connect the pneumatic cylinders to the air supply unit;
• a control unit which manages the operations of the air supply apparatus and the pneumatic cylinders,
**characterized in that** said pneumatic actuators (3) are actuated instantaneously, individually or in cluster group, or in a mixed manner individually and in cluster, using a touch interface installable on wireless/wired devices.

2. Pneumatic massage bed as claimed in claim 1 **characterized in that** each cluster group consists of at least of two pneumatic actuators (3) provided at the bottom of a supply line (5) of pressurized air in common which determines the simultaneous actuation.

3. Pneumatic massage bed as claimed in claim 1 **characterized in that** each cluster group consists of at least three pneumatic actuators (3) arranged at the vertices of polygonal or broken lines shapes, or sorted in segments, said types of layouts being arranged on the massage bed in an homogeneous or mixed manner, even with individual actuators, and along horizontal, vertical or diagonal directions.

4. Pneumatic massage bed as claimed in the previous claims **characterized in that** said single effect pneumatic actuators (3) are pistons, each provided with a single chamber, consisting of a hollow cylindrical body (4), within which slides a stem (5), provided at the bottom with a movable head plunger (6) supporting a soft seal (7), and on the top with a mobile head (8) connected, through suitable spherical jointing, means, for example screw (19), with a joint (9) made of rubber, said movable head (8) consisting of a flat circular surface (10) for the application, for example by gluing, of discs (11) of material suitable for heat transfer.

5. Pneumatic massage bed as claimed in claim 4 **characterized in that** the hollow cylindrical body (4) of the piston integrates at the bottom two fast connectors (12), hot plugged, for the connection of suitable compressed air tubes.

6. Pneumatic massage bed as claimed in claims 4 and 5 **characterized in that** on the hollow cylindrical body (4) of the piston are screwed, by means of threads (15, 16), two ferrule, an upper (13) and a lower (14), for fixing said piston on panels of varying thickness, said ferrule being provided with grooves (17) which prevent unscrewing.

7. Pneumatic massage bed as claimed in claim 6 **characterized in that** the upper ferrule (13), acting as a cap for the cylindrical body (4) of the piston, is provided with a through hole (18) for the sliding of the stem (5) of the piston and is designed to house a spring (20) with the function of the damper of the final phase of extension of the stem.

8. Massage bed as claimed in claims 3 to 7 **characterized in that** above the pistons is provided the application of a thin layer, for example, foam bamboo, or alternatively latex, natural polymers, viscoelastic materials, which provide a flat surface, similar to that of a mat, for the support of a user lying down.

9. Pneumatic massage bed as claimed in claims 3 to 8 **characterized in that** the bottom of the pistons are disposed infrared sources equipped with discs in stone, for example jade or tourmaline, for the heating of the mat which, provided with though holes, allows the exchange of heat between the massage bed and the user.

10. A system for operating the massage bed **characterized in that** it is activated and controlled via a dedicated touch interface, usable on wireless, or alternatively wired, devices, in an automatic or custom manner, effecting:
• linear waves, triggered by typing a vector, on the screen of the mobile device that represents the massage bed, the massage with linear waves being implemented in the direction indicated by the vector and having a speed proportional to the typing speed of the vector, and/or
• circular waves triggered by touch, on the screen of said mobile device, the massage with circular wave being carried out in concentric circles from the trigger point.
